# EUROPEAN PATENT APPLICATION

(11) **EP 2 944 292 A1**
(43) Date of publication of application: **18.11.2015**
(21) Application number: 13870709.6
(22) Date of filing: 08.01.2013
(51) Int. Cl.: A61F 2/82

(54) **STENT DELIVERY DEVICE**

(71) Applicant: Terumo Kabushiki Kaisha, Tokyo 151-0072 (JP)
(72) Inventor: KITAOKA, Takashi, Ashigarakami-gun Kanagawa 259-0151 (JP)
(74) Representative: Casalonga
(86) International application number: PCT/JP2013/050127
(87) International publication number: WO 2014/109003

(57) **Abstract**

A stent delivery device (10) according to the present invention includes a stent (12) arranged in a self-expandable state between a shaft (14) and an inner sheath (18). In a state where an outer sheath (16) is moved rearward and an expansion limiting portion (34) is exposed outward, the expansion limiting portion (34) formed in a distal portion of the inner sheath (18) limits expansion of the stent (12) so that an outer diameter of a distal end of the stent (12) becomes a first diameter (D1) which is larger than an inner diameter (Da) of the outer sheath (16), and in a state where the inner sheath (18) is moved rearward and the stent (12) is exposed outward, the stent (12) expands so that the outer diameter of the distal end of the stent (12) becomes a second diameter (D2) which is larger than the first diameter (D1).

## Description

### Technical Field

The present invention relates to a stent delivery device for causing a stent to indwell in a living body lumen.

### Background Art

Conventionally, in order to improve a lesion (stenosis or occluded portion) appearing inside a living body lumen such as blood vessels, biliary ducts, bronchial tubes, esophagi, urethrae, and the like, a stent delivery device for causing a stent to indwell in the lesion has been widely used.

For example, JP-T-2008-529719 discloses a stent delivery device which includes an inner sheath arranged relative to be movable to the shaft in an axial direction, an outer sheath disposed to be slidable relative to the inner sheath in the axial direction, and a stent received in a self-expandable state between the shaft and the inner sheath, and in which multiple strip-shaped tail portions separated by multiple slits are formed on a distal side of the inner sheath for receiving the stent.

The stent delivery device causes the outer sheath to move rearward relative to the inner sheath, thereby expanding the stent so that the multiple tail portions are pressed against an inner wall surface of the living body lumen. Then, the inner sheath is caused to move rearward relative to the shaft, thereby pulling out the multiple tail portions from a portion between the stent and the inner wall surface of the living body lumen. In this manner, it is possible to minimize damage to coating of the stent when the stent is deployed into the living body lumen and is caused to indwell in the living body lumen.

### Summary of Invention

Incidentally, when a self-expandable stent is caused to indwell at an indwelling-targeted portion in a living body lumen, if a sheath is moved rearward, the stent simultaneously expands all at once and indwells at an inner wall surface of the living body lumen. Consequently, the stent is likely to be misaligned with the indwelling-targeted portion as a separated distance is farther between the stent in a received state and the indwelling-targeted portion.

The stent delivery device according to the above-described prior art has the tail portion of the inner sheath which is arranged between the stent and the outer sheath. However, the stent delivery device aims to minimize damage to the coating of the stent. Thus, if the outer sheath is moved rearward, the stent simultaneously expands all at once to reach a position of substantially coming into contact with an inner wall surface of the living body lumen. Consequently, it is not possible to prevent the stent from being misaligned with the indwelling-targeted portion. In addition, after the stent expands, when the tail portion located between the inner wall surface of the living body lumen and the stent is pulled out, there is a possibility that the stent may be misaligned with the indwelling-targeted portion of the living body lumen.

The present invention is made in view of the above-described problem, and an object thereof is to provide a stent delivery device which can prevent a stent from being misaligned with an indwelling-targeted portion, when the self-expandable stent is caused to expand and indwell at the indwelling-targeted portion of a living body lumen.

[1] A stent delivery device according to the present invention includes a shaft, a tubular inner sheath that is arranged on an outer surface side of the shaft so as to be movable relative to the shaft along an axial direction, a tubular outer sheath that is arranged on an outer surface side of the inner sheath so as to be slidable relative to the inner sheath along the axial direction, and a stent that is received in a self-expandable state between the shaft and the inner sheath. A distal portion of the inner sheath has an expansion limiting portion which is configured to be more flexible than the outer sheath and by which at least a distal end of the stent is received. In a state where the outer sheath is moved rearward and the expansion limiting portion is exposed outward from the outer sheath, the expansion limiting portion limits expansion of the stent so that an outer diameter of the distal end of the stent becomes a first diameter which is larger than an inner diameter of the outer sheath. In a state where the inner sheath is moved rearward and the stent is exposed outward from the inner sheath, the stent expands so that the outer diameter of the distal end of the stent becomes a second diameter which is larger than the first diameter.
   According to the stent delivery device in the present invention, if the outer sheath is moved rearward and the expansion limiting portion of the inner sheath is exposed outward from the outer sheath, the stent expands so that the outer diameter of the distal end of the stent becomes the first diameter which is larger than the inner diameter of the outer sheath (first expansion). Then, if the inner sheath is moved rearward and the distal end of the stent is exposed outward from the inner sheath, the stent expands so that the outer diameter of the distal end of the stent becomes the second diameter which is larger than the first diameter (second expansion). That is, the stent can expand at two steps in the living body lumen. In this manner, after the stent is delivered to an indwelling-targeted portion of the living body lumen, it is possible to finely adjust a position of the stent by maintaining a state where the stent is subjected to the first expansion so as to shorten a separated distance between the distal end of the stent and the indwelling-targeted portion. Subsequently, the stent is subjected to the second expansion. In this manner, it is possible to reliably cause the stent to indwell at the indwelling-targeted portion. Therefore, when the self-expandable stent is caused to expand and indwell at the indwelling-targeted portion of the living body lumen, it is possible to prevent the stent from being misaligned with the indwelling-targeted portion.
[2] In the above-described stent delivery device, the inner sheath may have an inner sheath main body which is disposed on a proximal side relative to the expansion limiting portion and which has greater rigidity than that of the expansion limiting portion. When a total length of the expansion limiting portion is set to L, a total length of the stent in a received state is set to L1, and an outer diameter of the stent in a natural state is set to D, an equation of L≤L1-D/2 may be satisfied.
   Here, the stent in the natural state means a stent left as it is without having any restraints at all. According to this configuration, the equation of L≤L1-D/2 is satisfied. Therefore, when the distal end of the stent is subjected to the second expansion, it is possible to locate the proximal end of the stent inside the inner sheath main body which has the greater rigidity than that of the expansion limiting portion. In this manner, the distal end of the stent can be subjected to the second expansion so as to have the second diameter in a state where the proximal end of the stent is pressed against and held by the inner surface of the inner sheath main body by using a relatively strong restraining force. Accordingly, the distal end of the stent reliably comes into contact with the indwelling-targeted portion during the second expansion. Therefore, it is possible to preferably prevent the distal end of the stent from being misaligned with the indwelling-targeted portion in the axial direction. In addition, after the distal end of the stent is indwelled at the indwelling-targeted portion, the movement of the stent with respect to the indwelling-targeted portion is limited. Therefore, there is no possibility that the stent may be misaligned with the indwelling-targeted portion during the second expansion of the proximal side of the stent.
[3] In the above-described stent delivery device, flexibility of the expansion limiting portion may increase continuously or stepwisely toward a distal end.
   According to this configuration, the flexibility of the expansion limiting portion increases continuously or stepwisely toward the distal end. Therefore, in a state where the stent is subjected to the first expansion, the distal end within the stent can be moved closest to the indwelling-targeted portion. In this manner, it is possible to efficiently prevent the stent from being misaligned with the indwelling-targeted portion in the axial direction during the second expansion of the distal end of the stent.
[4] The above-described stent delivery device may further include a stopper portion which is disposed on the shaft so as to limit displacement along the axial direction of the stent.
   According to this configuration, the stopper portion is disposed on the shaft portion. Therefore, it is possible to preferably prevent the stent from being misaligned with the indwelling-targeted portion during the expansion of the stent.
[5] In the above-described stent delivery device, the stopper portion may be located inside the inner sheath main body and may engage with the stent in a state where the distal end of the stent is exposed from the expansion limiting portion.
   According to this configuration, in a state where the distal end of the stent is exposed from the expansion limiting portion, the stopper portion is located inside the inner sheath main body and engages with the stent. Therefore, it is possible to reliably prevent the stent from being misaligned with the indwelling-targeted portion in the axial direction during the second expansion of the distal end of the stent.
[6] In the above-described stent delivery device, the stopper portion may be in contact with a proximal end of the stent in a state of being fixed to the shaft.
   According to this configuration, the stopper portion is in contact with the proximal end of the stent in a state of being fixed to the shaft. Therefore, when the inner sheath is moved rearward relative to the shaft, it is possible to reliably prevent the stent from being misaligned to the proximal side with the indwelling-targeted portion.
[7] In the above-described stent delivery device, the stopper portion may have a locking portion which extends in a radial direction of the shaft and which is arranged to be inserted into a gap of struts of the stent.
   According to this configuration, the locking portion of the stopper portion is inserted into the gap of the struts of the stent. Therefore, when the inner sheath is moved rearward relative to the shaft, it is possible to reliably prevent the stent from being misaligned with the indwelling-targeted portion in the axial direction. The stent expands so that each of the struts slips out from the locking portion of the stopper portion. Therefore, there is no possibility that the stopper portion may interfere with the expansion of the stent in the radial direction.

### Brief Description of Drawings

[Fig. 1] Fig. 1 is a partially omitted vertical cross-sectional view of a stent delivery device according to an embodiment of the present invention.
[Fig. 2] Fig. 2 is an enlarged vertical cross-sectional view on a distal side of the stent delivery device in Fig. 1.
[Fig. 3] Fig. 3 is a cross-sectional view taken along line III-III in Fig. 2.
[Fig. 4] Fig. 4 is a side view of a stent in a natural state.
[Fig. 5] Fig. 5 is a partially omitted vertical cross-sectional view for illustrating a state where the stent in Fig. 1 is subjected to a first expansion.
[Fig. 6] Fig. 6 is a partially omitted vertical cross-sectional view for illustrating a state where a distal end of the stent in Fig. 5 is subjected to a second expansion.
[Fig. 7] Fig. 7 is a partially omitted vertical cross-sectional view for illustrating a state where the stent in Fig. 6 completely indwells at an indwelling-targeted portion of a living body lumen.
[Fig. 8] Fig. 8A is a partially omitted vertical cross-sectional view for illustrating a first modification example of an expansion limiting portion configuring the stent delivery device, and Fig. 8B is a partially omitted vertical cross-sectional view for illustrating a second modification example of the expansion limiting portion configuring the stent delivery device.
[Fig. 9] Fig. 9A is a partially omitted vertical cross-sectional view for illustrating a modification example of a stopper portion configuring the stent delivery device, and Fig. 9B is a cross-sectional view taken along line IXB-IXB in Fig. 9A.

### Description of Embodiments

Hereinafter, a stent delivery device according to the present embodiment will be described in detail with reference to preferred embodiments and the accompanying drawings.

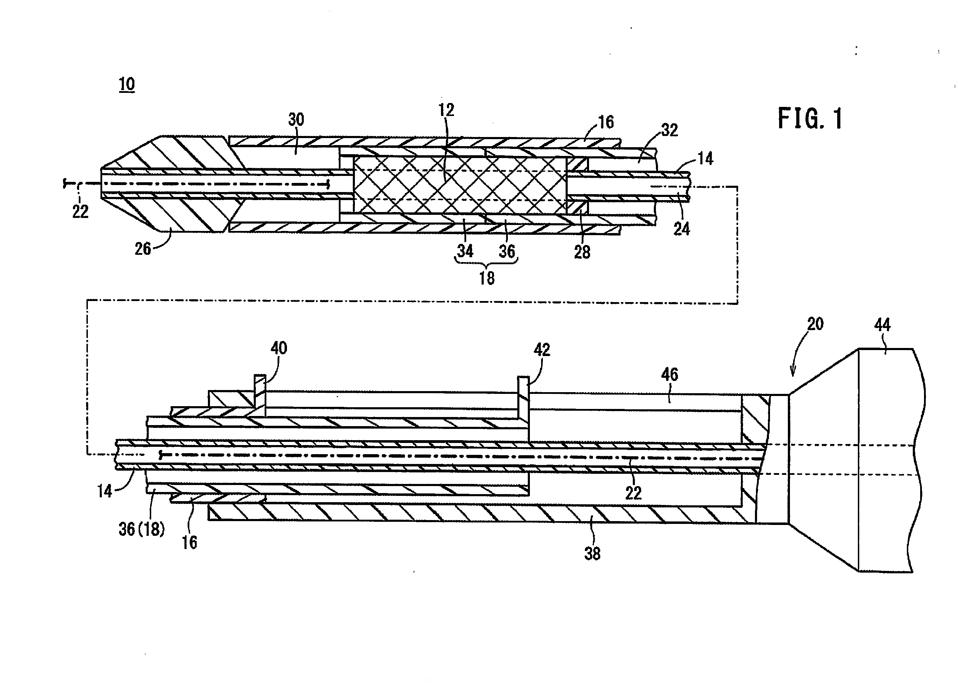

A stent delivery device 10 (hereinafter, simply referred to as a "delivery device 10") according to an embodiment of the present invention is a medical device for causing a stent 12 to indwell in a lesion in order to improve the lesion appearing inside a living body lumen such as blood vessels, biliary ducts, bronchial tubes, esophagi, urethrae, and the like. In the following description, the left side (stent 12 side) of the delivery device 10 in Fig. 1 is referred to as a "distal" side, and the right side (hub 44 side) of the delivery device 10 is referred to as a "proximal (rear)" side.

As illustrated in Fig. 1, the delivery device 10 includes a stent 12, an elongated shaft 14 having a thin diameter, an inner sheath 18 arranged on an outer surface side of the shaft 14 so as to be movable relative to the shaft 14 along an axial direction, an outer sheath 16 arranged on an outer surface side of the inner sheath 18 so as to be slidable relative to the inner sheath 18 along the axial direction, and a handle portion 20 configuring a proximal portion of the delivery device 10.

The stent 12 delivered into and caused to indwell in the living body lumen has a self-expandable function, and is received by a space formed between the shaft 14 and the inner sheath 18, thereby being brought into a state where the stent 12 is folded by the expansion being limited (contraction state, self-expandable state). The stent 12 can employ a configuration of axially arraying multiple frames in which a wire made of super-elastic alloy or the like such as Ti-Ni alloy and the like is formed in a ring shape or a Z-shape, or a configuration of braiding the wires made of the super-elastic alloy or the like in a mesh shape.

The shaft 14 is a flexible tubular member in which a guidewire lumen 24 into which a guidewire 22 is inserted is formed to have a through-hole over the total length thereof. The guidewire 22 is used to guide the delivery device 10 including the shaft 14 and the like to a lesion of the living body lumen. The shaft 14 extends to the proximal end of the handle portion 20 in a state of being arranged inside the outer sheath 16 so as to protrude beyond the distal end of the outer sheath 16.

A nose portion (nose cone) 26 is disposed in the distal end of the shaft 14. The nose portion 26 has a distal portion whose diameter decreases in a tapered shape toward the distal side, an intermediate portion whose diameter is constant, and a proximal portion whose diameter decreases in a tapered shape toward the proximal side. An outer diameter dimension of the intermediate portion of the nose portion 26 is set to be larger than an inner diameter of the outer sheath 16. That is, the nose portion 26 functions as a stopper for limiting movement toward the distal side of the outer sheath 16.

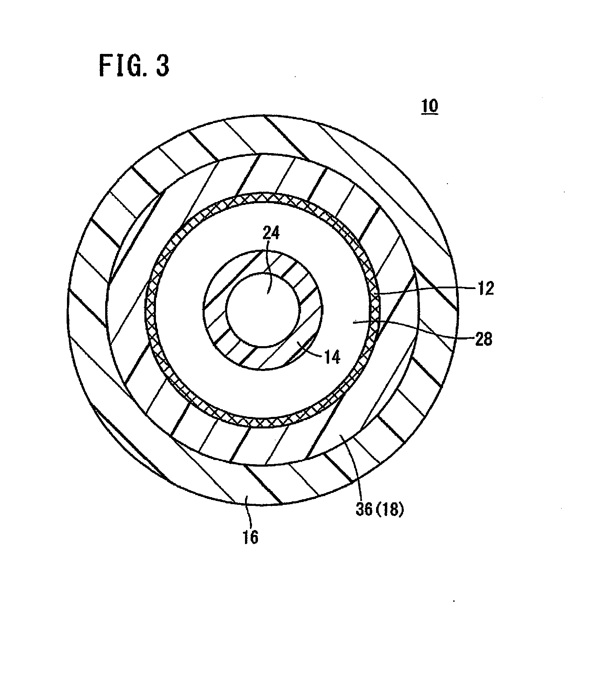

A stopper portion 28 for limiting displacement toward the proximal side of the stent 12 received inside the inner sheath 18 is disposed on the distal side of the shaft 14. The stopper portion 28 is fixedly attached to an outer surface of the shaft 14 in a state of being in contact with the proximal end of the stent 12. In addition, for example, the stopper portion 28 is configured to have an annular shape (refer to Fig. 3).

The outer sheath 16 is a flexible tubular member in which the lumen 30 enabling the inner sheath 18 to be arranged therein is formed to have a through-hole over the total length. The outer sheath 16 is arranged so as to be slidable relative to the inner sheath 18 in the axial direction, and has rigidity to such an extent so as not to be deformed due to an expansion force (spring force) of the stent 12 received inside the inner sheath 18.

A configuration material of the outer sheath 16 is not particularly limited, and may include polyolefin such as polyethylene, polypropylene, and the like, polyamide, polyester such as polyethylene terephthalate, and the like, fluorinated polymers such as PTFE, ETFE, and the like, thermoplastic elastomer such as polyamide elastomer, polyester elastomer, and the like, stainless steel, super-elastic metal, and the like.

The inner sheath 18 a flexible tubular member in which a lumen 32 enabling the shaft 14 to be arranged therein is formed to have a through-hole over the total length thereof. The inner sheath 18 is arranged inside the outer sheath 16 so that the distal end of the inner sheath 18 is located on the proximal side relative to the distal end of the outer sheath 16, and has an expansion limiting portion 34 configuring the distal portion of the inner sheath 18 and an inner sheath main body 36 disposed on the proximal side relative to the expansion limiting portion 34.

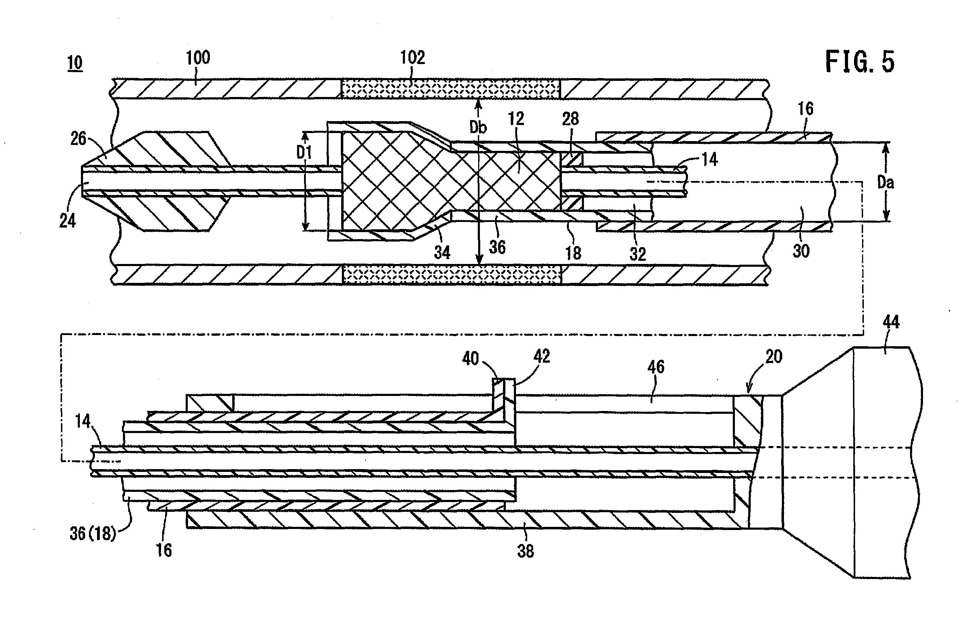

The expansion limiting portion 34 is used to limit the stent 12 so as not to expand all at once when the stent 12 is discharged (released) to the living body lumen, and at least the distal end of the stent 12 is received into the expansion limiting portion 34. In addition, the expansion limiting portion 34 is configured to be more flexible than the outer sheath 16. In other words, the expansion limiting portion 34 is configured to be expandable to have a first diameter D1 which is larger than an inner diameter Da of the outer sheath 16 by using an expansion force of the stent 12, in a state where the outer sheath 16 is moved rearward to the proximal side relative to the inner sheath 18 and the expansion limiting portion 34 is exposed outward from the outer sheath 16 (refer to Fig. 5).

For example, the expansion limiting portion 34 can be configured to include a material which is the same as that of the outer sheath 16. In this case, if the expansion limiting portion 34 is formed to be thinner than the outer sheath 16 (if the thickness of the expansion limiting portion 34 is formed to be thinner than the thickness of the outer sheath 16), the expansion limiting portion 34 can be more flexible than the outer sheath 16. As a matter of course, the expansion limiting portion 34 may be configured to be more flexible than the outer sheath 16 by changing the configuration material of the expansion limiting portion 34 and the configuration material of the outer sheath 16.

The inner sheath main body 36 is configured to have rigidity which is greater than that of the expansion limiting portion 34, and can be configured to include a material which is the same as that of the outer sheath 16, for example. In addition, for example, the inner sheath main body 36 can be configured to include a material which is the same as that of the expansion limiting portion 34. In this case, if the inner sheath main body 36 is formed to be thicker than the expansion limiting portion 34 (if the thickness of the inner sheath main body 36 is formed to be thicker than the thickness of the expansion limiting portion 34), the inner sheath main body 36 can be configured to have rigidity which is greater than that of the expansion limiting portion 34. In this case, the expansion limiting portion 34 and the inner sheath main body 36 may be configured integrally with each other. As a matter of course, the inner sheath main body 36 may be configured to have the rigidity which is greater than that of the expansion limiting portion 34 by changing the configuration material of the expansion limiting portion 34 and the configuration material of the inner sheath main body 36.

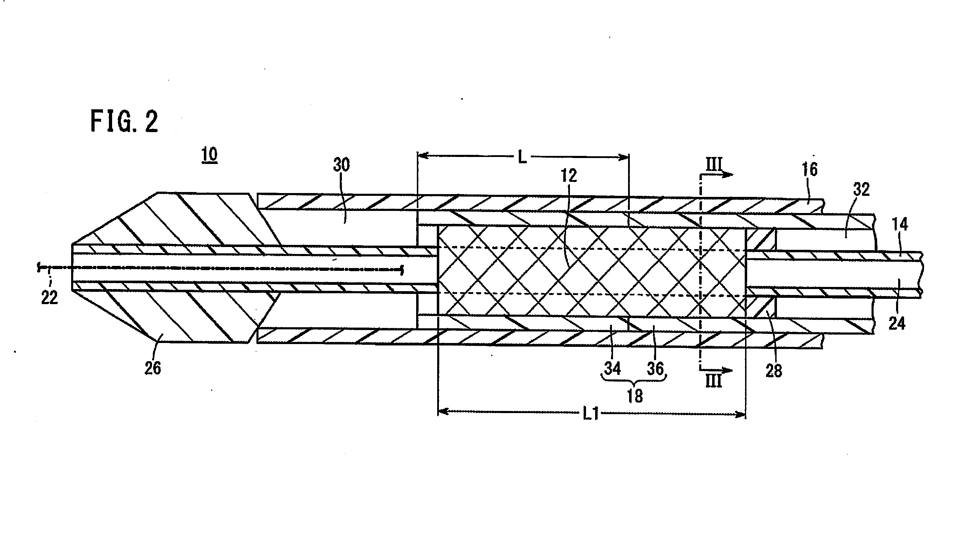

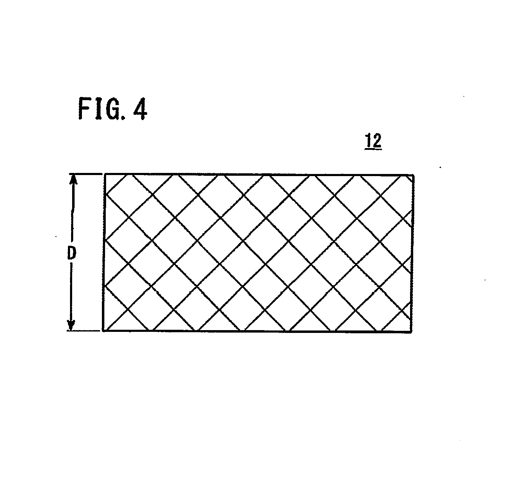

As illustrated in Figs. 2 and 4, the delivery device 10 according to the present embodiment satisfies an equation of L≤L1-D/2, when a total length of the expansion limiting portion 34 is set to L, a total length of the stent 12 in a received state is set to L1, and an outer diameter of the stent 12 in a natural state is set to D. Here, the stent 12 in the natural state means the stent 12 left as it is without having any restraints at all (non-restrained state). That is, the stent 12 in the natural state is in a state of being left as it is without any restraints at all in the axial direction and the radial direction.

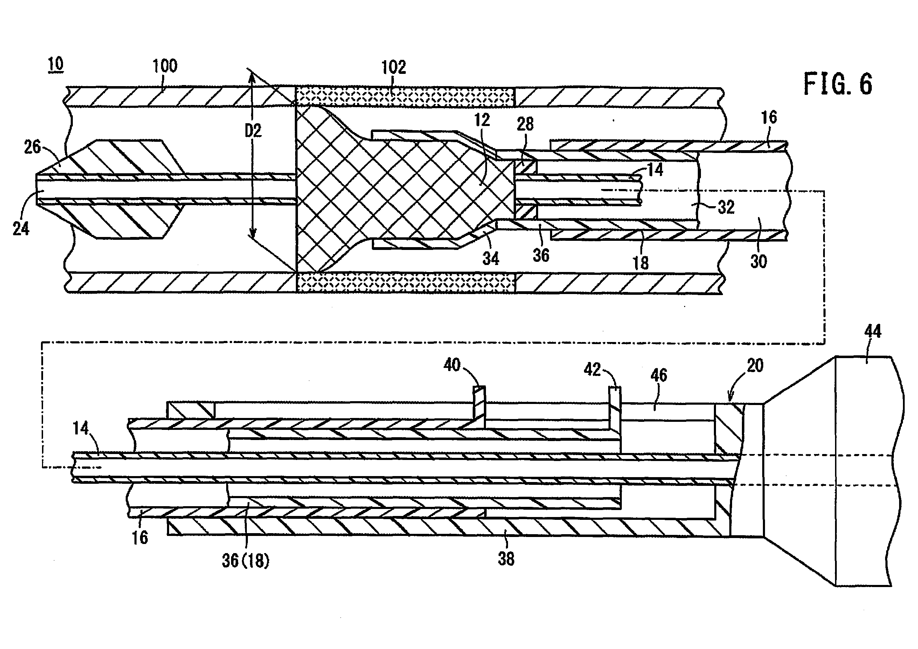

In the delivery device 10 satisfying the above-described equation, in the stent 12 in a received state, the proximal end of the stent 12 is located inside the inner sheath main body 36. Then, in a state where the inner sheath 18 is moved rearward to the proximal side relative to the shaft 14 and the distal end of the stent 12 is exposed outward from the expansion limiting portion 34, that is, in a state where the distal end of the stent 12 expands and indwells in the living body lumen, the proximal end of the stent 12 can be brought into contact with the stopper portion 28 inside the inner sheath main body 36 (refer to Fig. 6).

The handle portion 20 has a support portion 38 formed in a tubular shape, an operation pin 40 disposed at the proximal end of the outer sheath 16, an operation pin 42 disposed at the proximal end of the inner sheath main body 36, and a hub 44 fixed to the proximal end of the support portion 38. An opening 46 extending along the axial direction is formed on an outer surface of the support portion 38, and the respective operation pins 40 and 42 are exposed outward from the support portion 38 via the opening 46. In this manner, for example, a user or the like can move the outer sheath 16 and the inner sheath 18 independently in the axial direction by pinching the respective operation pins 40 and 42 with the user's fingers and causing the respective operation pins 40 and 42 to slide in the axial direction.

The delivery device 10 according to the present embodiment is basically configured as described above. Hereinafter, an operation and effect thereof will be described.

First, for example, a form of a lesion appearing in a blood vessel 100 (living body lumen) is identified by using an intravascular angiography method or an intravascular ultrasound diagnosis method. Next, for example, a Seldinger technique is used to percutaneously guide the guidewire 22 into the blood vessel 100 beforehand through the femoral region or the like. The guidewire 22 is inserted into the proximal end from the distal end of the guidewire lumen 24 of the shaft 14. The delivery device 10 including the shaft 14 and the like is guided into the aorta.

Then, under X-ray contrast using an X-ray opaque marker (not illustrated) disposed in the stent 12, the delivery device 10 is allowed to proceed until the stent 12 received in the distal side of the inner sheath 18 reaches an indwelling-targeted portion 102 of the blood vessel 100. Here, the indwelling-targeted portion 102 means a portion where the stent 12 in the blood vessel 100 is indwelled, and for example, means a portion where a lesion is present.

Subsequently, as illustrated in Fig. 5, a user or the like moves the outer sheath 16 to the proximal side relative to the inner sheath 18 by pinching the operation pin 40 with the user's fingers and pulling the operation pin 40 to the proximal side. Then, the expansion limiting portion 34 exposed outward from the outer sheath 16 is configured to be more reasonably flexible than the outer sheath 16. Accordingly, the stent 12 expands (first expansion) so that the outer diameter of the distal end of the stent 12 becomes the first diameter D1 which is larger than the inner diameter Da of the outer sheath 16.

At this time, the first diameter D1 is smaller than a hole diameter Db of the indwelling-targeted portion 102, and the inner sheath 18 is separated from an inner wall surface of the blood vessel 100. The first expansion of the stent 12 in this way can maintain a state where a separated distance is shortened between the distal end of the stent 12 and the indwelling-targeted portion 102. At this stage, the distal end of the outer sheath 16 is located on the proximal side relative to the expansion limiting portion 34, and the expansion limiting portion 34 is completely exposed outward from the outer sheath 16.

Next, a user or the like moves the delivery device 10 in the axial direction, thereby finely adjusting a position of the stent 12 so that the stent 12 reliably indwells at the indwelling-targeted portion 102. Subsequently, the user or the like pinches the operation pin 42 and pulls the operation pin 42 to the proximal side, thereby moving the inner sheath 18 rearward to the proximal side relative to the shaft 14 by a predetermined amount.

Then, as illustrated in Fig. 6, displacement toward the proximal side of the stent 12 is limited by the stopper portion 28. Accordingly, the distal end of the stent 12 is exposed outward from the inner sheath 18. Then, the distal end of the stent 12 exposed outward from the inner sheath 18 expands (second expansion) so as to have a second diameter D2 (D2 is the same as, or is slightly smaller than an outer diameter D of the stent 12 in a natural state) which is larger than the first diameter D1, and indwells at the indwelling-targeted portion 102. Here, the second diameter D2 is substantially as large as the hole diameter Db of the indwelling-targeted portion 102.

At this time, the proximal end of the stent 12 is in contact with the stopper portion 28 inside the inner sheath main body 36. Accordingly, it is possible to prevent the stent 12 from being misaligned with the indwelling-targeted portion 102 in the axial direction. Therefore, it is possible to reliably cause the distal end of the stent 12 to indwell at a predetermined position of the indwelling-targeted portion 102.

Subsequently, if the inner sheath 18 is further moved rearward to the proximal side relative to the shaft 14, the proximal end of the stent 12 is subjected to the first expansion and is separated from the stopper portion 28. However, since the distal end of the stent 12 has already indwelled at the indwelling-targeted site 102, there is no possibility that the stent 12 may be misaligned with the indwelling-targeted portion 102 in the axial direction.

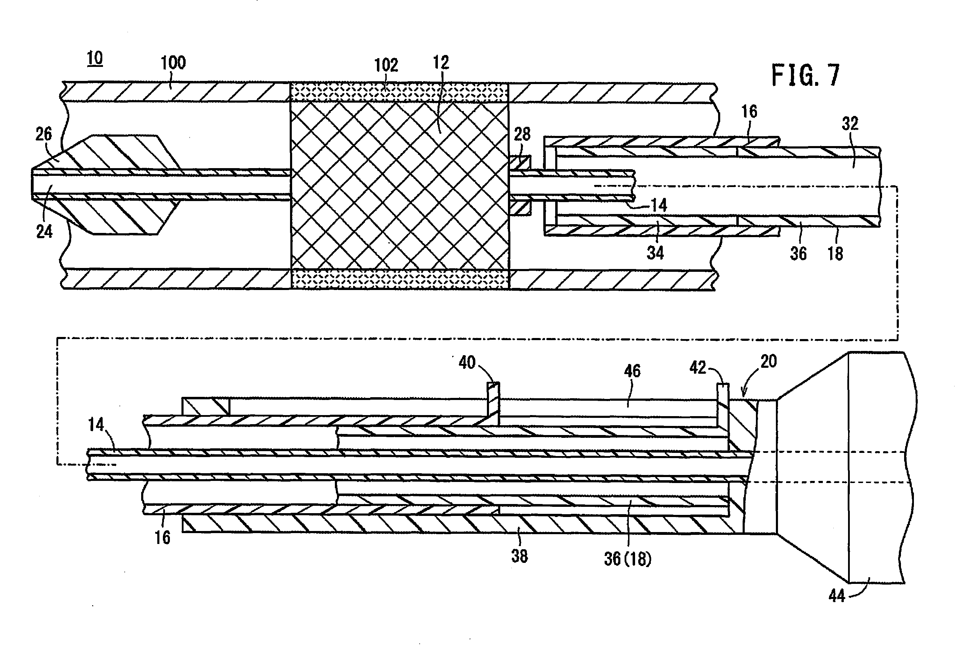

Then, the proximal end of the stent 12 is subjected to the second expansion, thereby causing all portions of the stent 12 to indwell at the indwelling-targeted portion 102 (refer to Fig. 7). At this time, the expansion limiting portion 34 is received inside the outer sheath 16 while contracting inward in the radial direction.

As described above, according to the delivery device 10 of the present embodiment, if the outer sheath 16 is moved rearward and the expansion limiting portion 34 of the inner sheath 18 is exposed outward from the outer sheath 16, the stent 12 is subjected to the first expansion so that the outer diameter of the distal end of the stent 12 becomes the first diameter D1 which is larger than the inner diameter Da of the outer sheath 16. If the inner sheath 18 is moved rearward and the distal end of the stent 12 is exposed outward from the inner sheath 18, the stent 12 is subjected to the second expansion so that the outer diameter of the distal end of the stent 12 becomes the second diameter D2 which is larger than the first diameter D1.

That is, this delivery device 10 enables the stent 12 to expand at two steps in the blood vessel 100. In this manner, after the stent 12 is delivered to the indwelling-targeted portion 102, it is possible to finely adjust a position of the stent 12 by maintaining a state where the stent 12 is subjected to the first expansion and the separated distance is shortened between the distal end of the stent 12 and the indwelling-targeted portion 102.

Subsequently, the stent 12 is subjected to the second expansion, thereby enabling the stent 12 to reliably indwell at the indwelling-targeted portion 102. Therefore, when the self-expandable stent 12 expands and indwells at the indwelling-targeted portion 102 in the blood vessel 100, it is possible to prevent the stent 12 from being misaligned with the indwelling-targeted portion 102.

The delivery device 10 according to the present embodiment is configured to satisfy the equation of L≤L1-D/2. Therefore, when the distal end of the stent 12 is subjected to the second expansion, the proximal end of the stent 12 can be positioned inside the inner sheath main body 36 whose rigidity is greater than the expansion limiting portion 34. In addition, during the second expansion, until the inner sheath 18 is moved rearward by a distance corresponding to a radius (D/2) of the stent 12 in at least a natural state, the proximal end of the stent 12 can be held in the inner surface of the inner sheath main body 36.

In this manner, the distal end of the stent 12 can be subjected to the second expansion so as to have the second diameter D2 in a state where the proximal end of the stent 12 is pressed against and held by the inner surface of the inner sheath main body 36 by using a relatively strong restraining force (in a state where there is no restraint of the blood vessel 100, if the inner sheath 18 is moved rearward by the distance corresponding to D/2, the stent 12 expands so as to have the outer diameter D). Accordingly, the distal end of the stent 12 reliably comes into contact with the indwelling-targeted portion 102 during the second expansion. Therefore, it is possible to preferably prevent the distal end of the stent 12 from being misaligned with the indwelling-targeted portion 102.

In addition, after the distal end of the stent 12 indwells at the indwelling-targeted portion 102, the movement of the stent 12 relative to the indwelling-targeted portion 102 is limited by the distal end of the stent 12. Therefore, there is no possibility that the stent 12 may be misaligned with the indwelling-targeted portion 102 during the second expansion of the proximal side of the stent 12.

The delivery device 10 according to the present embodiment has the stopper portion 28 for limiting displacement along the axial direction of the stent 12 disposed in the shaft 14. Therefore, when the stent 12 expands and indwells at the indwelling-targeted portion 102 of the blood vessel 100, it possible to preferably prevent the stent 12 from being misaligned with the indwelling-targeted portion 102.

In a state where the distal end of the stent 12 is exposed from the expansion limiting portion 34, the stopper portion 28 is positioned inside the inner sheath main body 36 and is in contact with (engages with) the proximal end of the stent 12. Therefore, it is possible to reliably prevent the stent 12 from being misaligned with the indwelling-targeted portion 102 during the second expansion of the distal end of the stent 12.

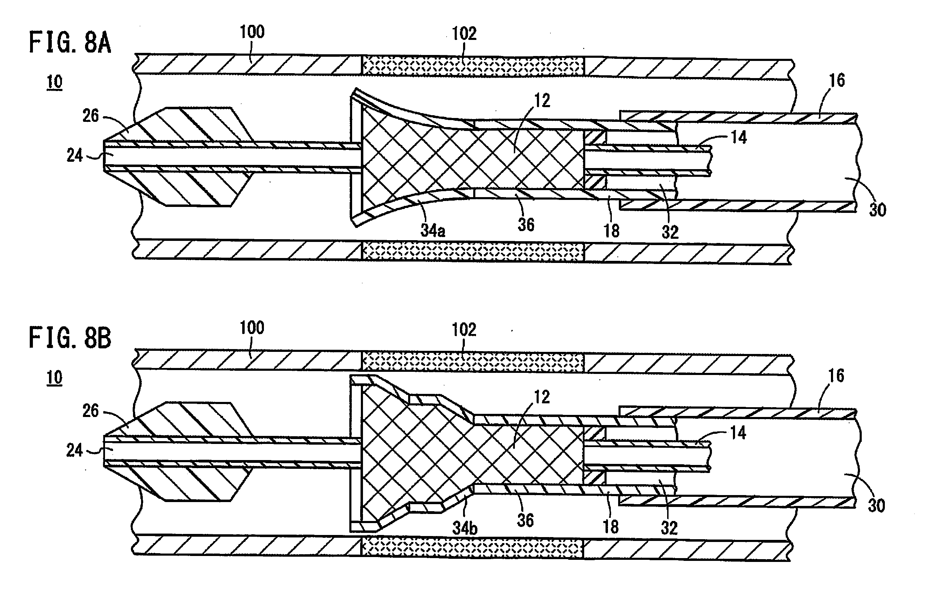

The present embodiment is not limited to the above-described configurations. For example, as illustrated in Fig. 8A, the delivery device 10 according to the present embodiment may have an expansion limiting portion 34a according to a first modification example instead of the expansion limiting portion 34.

The expansion limiting portion 34a is configured so that flexibility thereof continuously increases toward a distal end thereof. In this case, for example, a component ratio of the configuration material of the expansion limiting portion 34a is changed along the axial direction, and the flexibility can continuously increase toward the distal end of the expansion limiting portion 34a. In addition, the flexibility may continuously increase toward the distal end of the expansion limiting portion 34a by forming the expansion limiting portion 34a so as to be gradually thinner toward the distal end of the expansion limiting portion 34a.

According to the delivery device 10 including the expansion limiting portion 34a of the first modification example, it is possible to efficiently shorten a separated distance between the distal end of the stent 12 and the indwelling-targeted portion 102 in a state where the stent 12 is subjected to the first expansion. In this manner, it is possible to more reliably cause the distal end of the stent 12 to indwell at a predetermined position of the indwelling-targeted portion 102 during the second expansion of the distal end of the stent 12.

In addition, as illustrated in Fig. 8B, the delivery device 10 according to the present embodiment may have an expansion limiting portion 34b according to a second modification example instead of the expansion limiting portion 34. The expansion limiting portion 34b is configured so that flexibility thereof increases toward a distal end thereof stepwisely. Even in this case, it is possible to obtain an advantageous effect which is the same as that of the delivery device 10 having the expansion limiting portion 34a according to the first modification example.

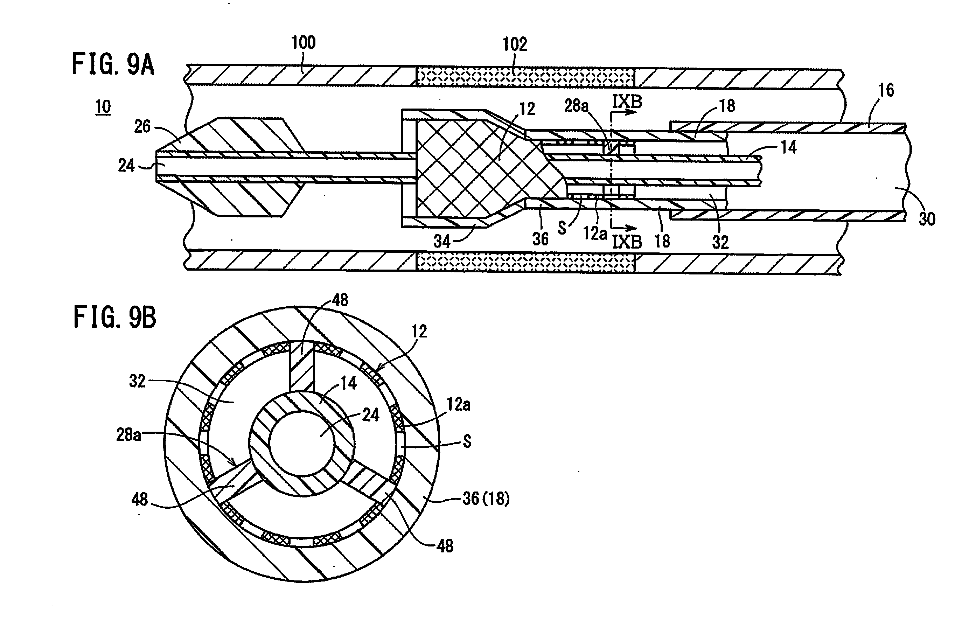

Furthermore, as illustrated in Figs. 9A and 9B, the delivery device 10 according to the present embodiment may have a stopper portion 28a according to a modification example instead of the stopper portion 28. The stopper portion 28a has multiple (for example, three) locking portions 48 which extend along the radial direction of the shaft 14 and which are arranged so as to be inserted into a gap S (mesh) between struts 12a of the stent 12. The respective locking portions 48 are configured to have a rod shape or a plate shape in a state of being fixedly attached to the outer surface of the shaft 14.

According to the delivery device 10 including the stopper portion 28a according to this modification example, the respective locking portions 48 of the stopper portion 28a are inserted into the gap S between the struts 12a of the stent 12. Therefore, when the inner sheath 18 is moved rearward to the proximal side relative to the shaft 14, it is possible to reliably prevent the stent 12 from being misaligned with the indwelling-targeted portion 102 in the axial direction.

In addition, the stent 12 expands so that the respective struts 12a slip out from the locking portions 48. Therefore, there is no possibility that the stopper portion 28a may interfere with the expansion of the stent 12. The stopper portion 28a according to the present modification example may have one locking portion 48, or may have four or more locking portions 48.

In the present embodiment, the delivery device 10 may be configured so that the stopper portion 28a according to the modification example is combined with the expansion limiting portion 34a according to the first modification example, or is combined with the expansion limiting portion 34b according to the second modification example.

Hitherto, the present invention has been described with reference to the preferred embodiments. However, without being limited to the above-described embodiments, the present invention can be modified in various ways within the scope not departing from the gist of the present invention, as a matter of course.

## Claims

1. A stent delivery device (10) comprising:
a shaft (14);
a tubular inner sheath (18) that is arranged on an outer surface side of the shaft (14) so as to be movable relative to the shaft (14) along an axial direction;
a tubular outer sheath (16) that is arranged on an outer surface side of the inner sheath (18) so as to be slidable relative to the inner sheath (18) along the axial direction; and
a stent (12) that is received in a self-expandable state between the shaft (14) and the inner sheath (18), wherein
a distal portion of the inner sheath (18) has an expansion limiting portion (34, 34a, 34b) which is configured to be more flexible than the outer sheath (16) and by which at least a distal end of the stent (12) is received,
in a state where the outer sheath (16) is moved rearward and the expansion limiting portion (34, 34a, 34b) is exposed outward from the outer sheath (16), the expansion limiting portion (34, 34a, 34b) limits expansion of the stent (12) so that an outer diameter of the distal end of the stent (12) becomes a first diameter (D1) which is larger than an inner diameter (Da) of the outer sheath (16), and
in a state where the inner sheath (18) is moved rearward and the stent (12) is exposed outward from the inner sheath (18), the stent (12) expands so that the outer diameter of the distal end of the stent (12) becomes a second diameter (D2) which is larger than the first diameter (D1).

2. The stent delivery device (10) according to Claim 1, wherein
the inner sheath (18) has an inner sheath main body (36) which is disposed on a proximal side relative to the expansion limiting portion (34, 34a, 34b) and which has greater rigidity than that of the expansion limiting portion (34, 34a, 34b), and
when a total length of the expansion limiting portion (34, 34a, 34b) is set to L, a total length of the stent (12) in a received state is set to L1, and an outer diameter of the stent (12) in a natural state is set to D, an equation of L≤L1-D/2 is satisfied.

3. The stent delivery device (10) according to Claim 1, wherein
flexibility of the expansion limiting portion (34a, 34b) increases continuously or stepwisely toward a distal end.

4. The stent delivery device (10) according to Claim 2, further comprising:
a stopper portion (28, 28a) which is disposed on the shaft (14) so as to limit displacement along the axial direction of the stent (12).

5. The stent delivery device (10) according to Claim 4, wherein
the stopper portion (28, 28a) is located inside the inner sheath main body (36) and engages with the stent (12) in a state where the distal end of the stent (12) is exposed from the expansion limiting portion (34, 34a, 34b).

6. The stent delivery device (10) according to Claim 5, wherein
the stopper portion (28) is in contact with a proximal end of the stent (12) in a state of being fixed to the shaft (14).

7. The stent delivery device (10) according to Claim 5, wherein
the stopper portion (28a) has a locking portion (48) which extends in a radial direction of the shaft (14) and which is arranged to be inserted into a gap (S) between struts (12a) of the stent (12).
